Europäisches Patentamt

European Patent Office

Office européen des brevets

(11) Numéro de publication: **0 278 864 B1**

# FASCICULE DE BREVET EUROPEEN

(45) Date de publication de fascicule du brevet: **17.06.92**

(51) Int. Cl.5: **A61N 1/365**, A61B 5/103, H03K 17/95

(21) Numéro de dépôt: **88400282.5**

(22) Date de dépôt: **08.02.88**

(54) Stimulateur cardiaque.

(30) Priorité: **09.02.87 FR 8701529**

(43) Date de publication de la demande:
**17.08.88 Bulletin 88/33**

(45) Mention de la délivrance du brevet:
**17.06.92 Bulletin 92/25**

(84) Etats contractants désignés:
**DE FR GB IT NL SE**

(56) Documents cités:
EP-A- 0 222 681
FR-A- 1 444 818
FR-A- 2 535 060
US-A- 3 518 997
US-A- 4 140 132

TRANSACTIONS ON BIO-MEDICAL ENGINEE-
RING, vol. BME 23, no. 2, mars 1976, pp
175-178; R.J. McPARTLAND et al.: "Activity
sensors for use in psycriatric evaluation".

MACHINE DESIGN THE ELECTRIC CONTROLS
BOOK ISSUE, vol. 34, no. 14, 16 juin 1962, pp
121-125; R.C. CROSSLEY: "Proximity switches".

(73) Titulaire: **ELA MEDICAL**
**98, rue Maurice Arnoux**
**F-92120 Montrouge(FR)**

(72) Inventeur: **BENS Jean-Luc**
**169, boulevard Victor-Hugo**
**62400 - BETHUNE(FR)**

(74) Mandataire: **Laget, Jean-Loup**
**Cabinet Pierre Loyer 77, rue Boissière**
**F-75116 Paris(FR)**

Rank Xerox (UK) Business Services

## Description

L'invention concerne un stimulateur cardiaque.

Il est connu d'utiliser un stimulateur cardiaque, soit pour les sujets atteints de tachycardie, soit pour les sujets atteints de bradycardie.

Dans le cas de la tachycardie, le stimulateur agit de la manière suivante : avant le stimulus naturel, il envoie un signal de dépolarisation qui définit une période réfractaire de durée déterminée, pour interrompre la propagation de l'influx, qui est circulaire dans le coeur ; après l'expiration de cette durée, la dépolarisation naturelle du coeur reprend jusqu'au déclenchement du cycle cardiaque naturel. Les stimulateurs connus gardent en mémoire la crise de tachycardie précédente et en déduisent la durée au bout de laquelle, à partir de la dernière contraction cardiaque, il faut envoyer le signal de dépolarisation pour éviter la fibrillation.

Ces stimulateurs présentent un inconvénient car ils ne savent pas distinguer la position, debout ou couchée, du sujet. Or la durée du cycle de tachycardie est différente suivant que le sujet est debout ou couché.

A titre d'exemple, alors qu'un cycle cardiaque naturel dure environ 800ms, un cycle de tachycardie peut durer 300ms pour un sujet couché et seulement 270ms pour le même sujet debout.

Si la dernière crise mémorisée a eu lieu alors que le sujet était couché, le stimulateur en déduit que le signal de dépolarisation doit être émis après une durée de 280 ms à partir de la dernière contraction cardiaque. Si maintenant le sujet est debout, avec un cycle de 270ms, le stimulateur devient inopérant car son signal de dépolarisation est émis trop tard (280ms) alors qu'il devrait être émis après 250ms environ pour tenir compte de la position debout du sujet.

Dans le cas de la bradycardie, le problème est tout-à-fait différent : il faut bien stimuler le déclenchement du cycle cardiaque à une fréquence déterminée. Les stimulateurs connus sont en général réglés indépendamment de la position du sujet ; cependant la stimulation peut être asservie au contrôle de l'oreillette, de l'intervalle Q-T entre les ondes Q et T du cardiogramme, de la température du sujet, de sa respiration ou de son activité, par exemple.

Ces stimulateurs ne savent pas distinguer directement la position, debout ou couchée, du sujet. Or, pendant son sommeil, le sujet n'a pas besoin d'avoir un rythme cardiaque aussi rapide qu'en période d'activité, ou nécessite un mode de fonctionnement différent du stimulateur. Par exemple, il est souhaitable pour certains sujets de passer d'un mode de fonctionnement à fréquence asservie à un mode séquentiel, ou à un mode de fonctionnement classique dit à la demande.

Le document EP-A-0 222 681 décrit un stimulateur cardiaque influençant le rythme de stimulation par un signal dépendant de l'effort du patient. Il comporte au moins deux enregistreurs de mesure pour des signaux dépendant de l'effort corporel, ainsi que des circuits pour traiter conjointement ces signaux de façon à obtenir un nouveau signal dépendant des deux signaux précédents. Il comporte également un interrupteur à mercure susceptible de reconnaître la position instantanée du patient (debout ou couché).

En outre, il mesure l'activité du patient au moyen d'un capteur à accéléromètres. Il réalise donc un véritable asservissement du rythme de stimulation à l'activité du patient, au moyen de composants et de circuits complexes.

Le document fait partie de l'état de la technique au sens de l'article 54(3) et (4) de la CBE et n'est opposable qu'au titre de la nouveauté, cette dernière résultant de la présence dans l'objet de la présente invention d'un oscillateur propre associé à chaque position du sujet.

Le document US-A-3 518 997 divulgue un stimulateur cardiaque tel que défin: dans le préambule de la revendication 1, dans lequel toutefois la commutation vers l'un ou l'autre des oscillateurs doit être commandée manuellement au moyen d'un interrupteur magnétique, selon le degré d'activé du sujet.

Un but de l'invention est de proposer un stimulateur cardiaque dont le cycle de fonctionnement s'adapte à la position du sujet.

Un autre but de l'invention est de proposer un capteur pour déterminer automatiquement et simplement la position du sujet afin d'y adapter le cycle de fonctionnement du stimulateur.

Un autre but encore de l'invention est de proposer un stimulateur cardiaque dont le mode de fonctionnement puisse être adapté à la position du sujet.

L'invention a pour objet un stimulateur cardiaque du type comportant : un premier oscillateur définissant un rythme de stimulation cardiaque correspondant à la position debout du sujet ; un deuxième oscillateur définissant un rythme de stimulation cardiaque correspondant à la position couchée du sujet ; un étage de sélection susceptible de délivrer des signaux de stimulation au rythme défini par l'un ou l'autre des oscillateurs, selon la position debout ou couchée du sujet, caractérisé en ce qu'il comporte en outre un capteur de la position debout ou couchée du sujet et un étage de décision susceptible, à partir des informations fournies par le capteur sur la position du sujet, de commander la commutation de l'étage de sélection vers l'un ou l'autre des oscillateurs.

Selon d'antes caractéristiques de l'invention:
  - le capteur de position est constitué par une sphère isolante dans laquelle est disposée une bille magnétique , et des capteurs de proximité placés à l'extérieur de la sphère et susceptibles de détecter la présence de la bille lorsqu'elle est à proximité de l'un desdits capteurs,

- des capteurs de proximité sont disposés autour de l'équateur de la sphère ,
- des capteurs de proximité sont disposés aux pôles de la sphère définissant l'axe vertical,
- les capteurs de proximité sont de type inductif.
- les axes de référence horizontal et vertical sont programmables, de manière non invasive, après implantation.
- l'étage de décision est susceptible, à partir des informations fournies par le capteur sur la position du sujet, de commander un changement de mode de fonctionnement du stimulateur.

D'autres caractéristiques ressortent de la description qui suit faite avec référence au dessin annexé sur lequel on peut voir :

Figure 1 : un schéma symbolique du circuit électronique du stimulateur cardiaque selon l'invention;

Figure 2 : une vue schématique d'un capteur de position du sujet utilisable dans le stimulateur cardiaque selon l'invention.

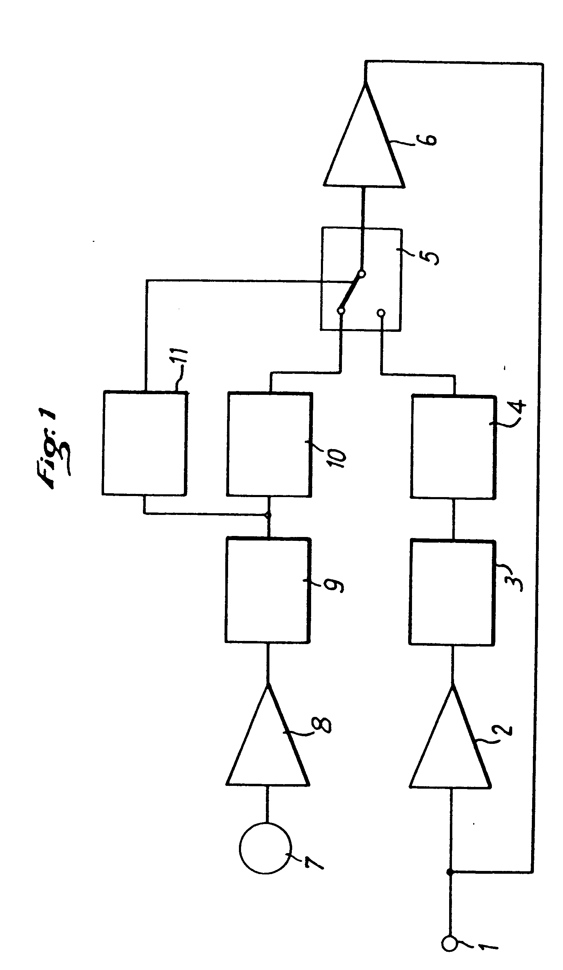

En se reportant à la figure 1, on voit que le stimulateur cardiaque selon l'invention comprend dans sa branche inférieure : une électrode 1 de détection de signal cardiaque et de stimulation, un amplificateur 2 du signal cardiaque, un circuit 3 définissant une période réfractaire pour éviter les déclenchements intempestifs ou accidentels dus à la physiologie ou à l'électronique, un oscillateur 4 définissant la période de stimulation lorsque le sujet est debout ou en activité, un étage de sélection 5 symbolisé par un commutateur à deux entrées et sortie unique, et un amplificateur de sortie 6 dont la sortie est appliquée à l'électrode 1.

Dans sa branche supérieure sur la figure 1, le stimulateur comprend en outre : un capteur 7 de la position, debout ou couchée , du sujet, un amplificateur 8, un circuit de temporisation 9 pour éviter les commutations trop fréquentes ou trop rapides, un oscillateur 10 définissant la période de stimulation lorsque le sujet est couché, et un étage de décision 11 commandé par la sortie du circuit de temporisation 9 et commandant la position du commutateur 5 en fonction de la position du sujet.

Les deux oscillateurs 4 et 10 sont reliés chacun à l'une des entrées du commutateur de l'étage de sélection 5. Ils sont en permanence en service, chacun à sa fréquence propre, mais le signal de sortie d'un seul d'entre eux est transmis à l'amplificateur de sortie 6.

L'électrode 1 détecte le signal naturel choisi comme paramètre de référence, par exemple un signal d'oreillette, ou de ventricule, ou autre.

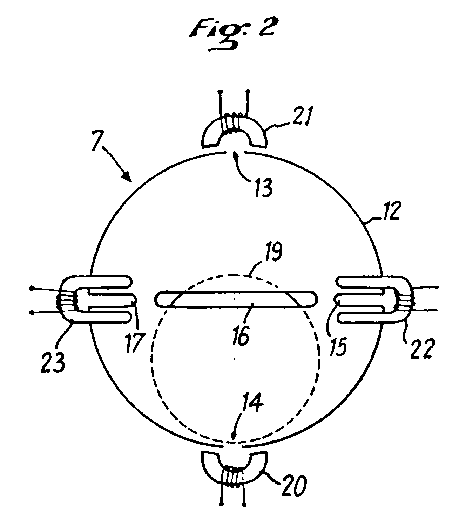

Le capteur de position 7 permet de définir la position, debout ou couchée, du sujet. Selon un mode de réalisation particulier, ce capteur est du type inductif, et il comporte (figure 2) : une sphère isolante 12, de faible épaisseur, munie à ses pôles d'orifices 13, 14, et à son équateur de lumières 15, 16, 17, 18 régulièrement réparties, dont trois seulement sont visibles sur la figure 2 ; à l'intérieur de la sphère 12 est logée une bille 19 en matériau magnétique, susceptible de rouler dans la sphère en fonction de la position du sujet. A l'extérieur de la sphère 12 sont disposés des capteurs de proximité, constitués par une armature et un enroulement relié à un circuit électronique non représenté parce que classique. Chacun de ces capteurs a une inductance propre dont la valeur varie lorsque la bille 19 est à proximité de l'armature. En fonction de la position du sujet, chacun des capteurs peut ainsi donner un premier signal en l'absence de bille et un second signal, différent du premier, si la bille est en face du capteur, dans la sphère 12.

Le capteur de position 7 est implanté avec l'axe des pôles disposé verticalement. L'un ou l'autre des capteurs de proximité 20 et 21 indique alors la position debout du sujet si la bille est en face de lui (cas de la figure 2). Si le sujet est en position couchée, c'est l'un des capteurs équatoriaux qui informe de la position du sujet. Deux seulement de ces capteurs équatoriaux, 22 et 23, sont représentés sur la figure 2. Leur armature respective est de préférence munie de pôles magnétiques larges pour détecter la présence de la bille en n'importe quel point d'une lumière 15-18. Il faut remarquer que les lumières ne sont qu'optionnelles et nullement indispensables, de même que les orifices polaires 13, 14. De même, on pourrait ne pas prévoir de capteurs polaires 20, 21 : en effet, si l'un des capteurs équatoriaux détecte la bille, alors le sujet est couché, mais si aucun ne la détecte, c'est que le sujet doit être en position redressée, ou en activité.

On peut également prévoir des paires de capteurs identiques ou non, disposés selon trois axes orthogonaux, et un amplificateur 8 programmable, de telle manière que, après implantation, on puisse programmer les axes de référence horizontal et vertical par une sélection appropriée des capteurs.

Pour en revenir à la figure 1, il faut noter que le circuit de temporisation 9 a une particularité. Ce circuit a pour rôle de retarder quelque peu la prise en compte, par l'étage de décision 11, du changement de position du sujet.

Lorsque le sujet était en position couchée, s'il change de position, il faut seulement une temporisation de quelques secondes pour le remettre au rythme cardiaque correspondant à une période d'activité, mais il faut quand même une petite temporisation pour éviter de modifier son rythme cardiaque alors qu'il ne fait que changer de position tout en restant couché. S'il était en position debout et qu'il passe en position couchée, il faut de préférence attendre quelques minutes avant de ralentir son rythme cardiaque.

Compte-tenu des signaux en provenance du capteur de position 7 et de la temporisation imposée par le circuit 9, l'étage de décision 11 commande le basculement de l'étage de sélection 5 vers l'oscillateur 4 définissant le rythme cardiaque pour la position debout (sujet en activité), ou vers l'oscillateur 10 définissant le rythme cardiaque pour la position couchée (repos ou sommeil).

Par ailleurs, on a remarqué que le seuil de stimulation, surtout au niveau de l'oreillette, augmente lors du passage de la position couchée à la position debout. Pour pallier cette augmentation de seuil liée au passage en clinostatisme, l'invention prévoit une augmentation automatique de l'amplitude du signal de sortie du stimulateur, avec éventuellement un allongement de la durée de ce signal.

On voit que le stimulateur cardiaque selon l'invention s'applique aussi bien au cas de la tachycardie qu'au cas de la bradycardie. La distinction entre les deux cas n'intervient qu'au niveau des oscillateurs 4 et 10 dont les fréquences doivent être adaptées soit au cas de la tachycardie, soit au cas de la bradycardie.

Ce stimulateur pourra être multiprogrammable par ailleurs avec un suiveur de seuil permettant de calculer de façon externe et chronique le seuil endocavitaire de stimulation. Il pourra enfin être appliqué soit au niveau de l'oreillette, en particulier dans le syndrome de bradycardie-tachycardie sinusale, soit au niveau du ventricule. Cette adaptation du type de fonctionnement du stimulateur cardiaque à la position du sujet présente en quelque sorte un volant d'inertie permettant de s'adapter à une position donnée, procubitus ou orthostatisme avec un rôle d'amortisseur lors des changements de position.

Enifn, le stimulateur cardiaque selon l'invention est susceptible, en fonction de la position debout ou couchée du sujet, de commander un changement de mode de fonctionnement du stimulateur, pour le faire passer d'un mode à fréquence asservie à un mode de stimulation à la demande.

## Revendications

1. Stimulateur cardiaque du type comportant : un premier oscillateur (4) définissant un rythme de stimulation cardiaque correspondant à la position debout du sujet ; un deuxième oscillateur (10) définissant un rythme de stimulation cardiaque correspondant à la position couchée du sujet ; un étage de sélection (5) susceptible de délivrer des signaux de stimulation au rythme défini par l'un ou l'autre des oscillateurs , caractérisé en ce qu'il comporte en outre : un capteur (7) de la position debout ou couchée du sujet et un étage de décision (11) susceptible à partir des informations fournies par le capteur (7) sur la position du sujet, de commander la commutation de l'étage de sélection (5) vers l'un ou l'autre des oscillateurs.

2. Stimulateur selon la revendication 1, caractérisé en ce que le capteur de position (7) est constitué par une sphère isolante (12) dans laquelle est disposée une bille magnétique (19), et des capteurs de proximité placés à l'extérieur de la sphère et susceptible de détecter la présence de la bille lorsqu'elle est à proximité de l'un desdits capteurs.

3. Stimulateur selon la revendication 2, caractérisé en ce que des capteurs de proximité (22, 23) sont disposés autour de l'équateur de la sphère (12).

4. Stimulateur selon la revendication 2, caractérisé en ce que des capteurs de proximité (20, 21) sont disposés aux pôles de la sphère (12) définissant l'axe vertical.

5. Stimulateur selon la revendication 2, caractérisé en ce que les capteurs de proximité sont de type inductif.

6. Stimulateur selon la revendication 2, caractérisé en ce que les axes de référence horizontal et vertical sont programmables, de manière non invasive, après implantation.

7. Stimulateur selon la revendication 1, caractérisé en ce que l'étage de décision (11) est susceptible, à partir des informations fournies par le capteur (7) sur la position du sujet, de commander un changement de mode de fonctionnement du stimulateur.

## Claims

1. Cardiac stimulator of the type comprising: a first oscillator (4) defining a rhythm for cardiac stimulation corresponding to the standing position of the patient; a second oscillator (10) defining a rhythm for cardiac stimulation corresponding to the prone position of the patient; a selection stage (5) capable of delivering stimulation signals at the rhythm defined by one or other of the oscillators, characterised in that it also comprises; a sensor (7) for sensing the standing or prone position of the patient and a decision stage (11) capable, with the information about the position of the patient provided by the sensor (7), of controlling the switching of the selection stage (5) to one or other of the oscillators.

2. Stimulator according to Claim 1, characterised in that the sensor (7) for sensing the patient's position is composed of an insulating sphere (12) containing a magnetic head (19) and close-range sensors placed outside the sphere and capable of detecting the presence of the head when it is close to one of the sensors.

3. Stimulator according to Claim 2, characterised in that close-range sensors (22, 23) are placed around the equator of the sphere (12).

4. Stimulator according to Claim 2, characterised in that close-range sensors (20,21) are placed at the poles defining the vertical axis of the sphere (12).

5. Stimulator according to Claim 2, characterised in that the close-range sensors are of the inductive type.

6. Stimulator according to Claim 2, characterised in that the horizontal and vertical axes of reference can be programmed non-invasively after implantation.

7. Stimulator according to Claim 1, characterised in that the decision stage (11) is capable, with the information about the patient's position provided by the sensor (7), of changing the operating mode of the stimulator.

**Patentansprüche**

1. Herzstimulationsgerät mit einem ersten Oszillator (4) zum Definieren eines Herzstimulationsrhythmus, der dem einer aufgerichteten Person entspricht, einem zweiten Oszillator (10) zum Definieren eines Herzstimulationsrhythmus, der dem einer liegenden Person entspricht, einer Auswahlstufe (5) zum Bereitstellen von Stimulationssignalen entsprechend dem Rhythmus eines der beiden Oszillatoren, gekennzeichnet durch einen Positionsfühler (7) zum Erfassen der Lage einer Person und einer Entscheidungsstufe (11), die anhand der Informationen des Positionsfühlers (7) entscheidet, ob die Person aufgerichtet ist oder liegt, und dementsprechend die Auswahlstufe (5) auf einen der beiden Oszillatoren schaltet.

2. Herzstimulationsgerät nach Anspruch 1, dadurch gekennzeichnet, daß der Positionsfühler (7) aus einer isolierten Kugel (12) besteht, in der sich eine magnetische Kugel (19) befindet, wobei außerhalb der isolierten Kugel Näherungsfühler angebracht sind, die das Vorhandensein der magnetischen Kugel anzeigen, wenn diese in die Nähe eines der Näherungsfühler kommt.

3. Herzstimulationsgerät nach Anspruch 2, dadurch gekennzeichnet, daß Näherungsfühler (22, 23) um den Äquator der isolierten Kugel (12) angebracht sind.

4. Herzstimulationsgerät nach Anspruch 2, dadurch gekennzeichnet, daß Näherungsfühler (20, 21) an den Polen der isolierten Kugel (12) angebracht sind, wodurch die Vertikalachse definiert ist.

5. Herzstimulationsgerät nach Anspruch 2, dadurch gekennzeichnet, daß die Näherungsfühler Induktionsfühler sind.

6. Herzstimulationsgerät nach Anspruch 2, dadurch gekennzeichnet, daß nach der Implantation ohne weiteren Eingriff horizontale und vertikale Bezugsachsen programmierbar sind.

7. Herzstimulationsgerät nach Anspruch 1, dadurch gekennzeichnet, daß die Entscheidungsstufe (11), anhand der Informationen von dem Positionsfühler (7) eine Änderung im Betriebsablauf des Herzstimulatiomsgeräts bewirkt.

Fig. 1

Fig. 2